# EUROPEAN PATENT APPLICATION

(11) **EP 0 898 950 A1**
(43) Date of publication of application: **03.03.1999**
(21) Application number: 97114841.6
(22) Date of filing: 27.08.1997
(51) Int. Cl.: A61F 13/15

(54) **Disposable absorbent article having a dilatant behaviour**

(71) Applicant: THE PROCTER & GAMBLE COMPANY, Cincinnati, Ohio 45202 (US)
(72) Inventor: Gagliardi, Ivano, 65123 Pescara (IT); Guarracino, Mario, 64028 Silvi Marina (IT)
(74) Representative: Hirsch, Uwe Thomas

(57) **Abstract**

A disposable absorbent article (20) omprising a liquid pervious topsheet (30), a backsheet (50) joined to the topsheet (30) and an absorbent core (60) intermediate the backsheet (50) and the topsheet (30). The disposable absorbent article (20) has an increased capacity to withstand bunching and twisting in use, since it shows a resistance to deformation that increases with the amount of the deformation.

## Description

### FIELD OF THE INVENTION

The present invention relates to disposable absorbent articles. Disposable absorbent articles are designed to be worn externally of the body by a user and to receive fluids discharged from the body, such as disposable absorbent pantiliners, sanitary napkins, catamenials, and incontinence inserts. Particularly the present invention relates to such articles that exhibit an increased comfort for the user by showing a dilatant behaviour, due to the use of a particulate material in such articles.

### BACKGROUND OF THE INVENTION

In their basic form, disposable absorbent articles comprise an absorbent core interposed between a pervious body-contacting element (alternatively referred to as a topsheet or an overwrap) and an impervious protective barrier (alternatively referred to as a backsheet). The absorbent core is, of course, intended to receive and contain the fluids discharged from the body. The body-contacting element is intended to provide comfortable and dry-feeling contact with body surfaces while allowing free passage of fluids therethrough into the absorbent element. The protective barrier is intended to prevent the fluids which are expelled or which escape from the absorbent element from soiling the user's garments.

Improved comfort for the user has been since long recognized as a need for this category of products. Thin disposable absorbent articles, typically featuring a thickness of less than 5 mm, are for example appreciated since they are more comfortable and discreet. Improvements in comfort in disposable absorbent articles, particularly in preferred thin articles, have been usually achieved at the expense of the level of protection of said articles. A softer article, that may be preferred in terms of comfort, can in fact lead to twisting or bunching in use under the forces exerted on it by the user's body and, possibly, by the undergarment, during the movements normally experienced in the wearing time. This tendency is even more pronounced in the wet state of the article, when structures of disposable absorbent articles usually loose part of their original strength and stability and are less capable of withstanding the stresses occurring in use. The twisting and bunching is referred to as "roping" since a cylindrical profile can be imparted to the absorbent article. The roping effect is detrimental because the absorbent article is less capable of absorbing body fluid that contact its upper surface. For example, the fluid discharged from the vagina has in fact a tendency to run off the roped napkin before it can be absorbed and, therefore, the fluid leaks onto the undergarment. This run-off becomes significant during periods of heavy flow. Moreover, the roping causes a reduction in the useful area of the absorbent article. Besides being uncomfortable to the user, a twisted absorbent article has a smaller strike zone to catch body fluids and thus is less effective in intercepting them. This also results in more frequent leakage.

Providing a disposable absorbent article with a stiffer and/or more resilient structure may solve on one hand the roping problem, since the article is then more capable of resisting to the deformation induced by the user's movements and/or of recovering its original optimal configuration after deformation. This was suggested for example in US 4,217,901, where the use of superabsorbent particles for providing increased crush resistance was disclosed. This poses on the other hand a comfort issue since the higher stiffness and/or resiliency make in any case the article resist with an approximately constant force to the wearer's movements, which in order to prevent bunching and twisting is typically sufficiently high to cause discomfort.

It has now been found that the use of suitable particulate materials in a disposable absorbent article is beneficial as far as comfort is concerned since it can provide the article with a so called dilatant behaviour, or force dependant resistance.

Particularly preferred particulate materials suitable to provide dilatancy are some of the materials that are commonly used in disposable absorbent articles as odour control agents.

Odour control has been another aspect of absorbent articles that has been under investigation for many years. Many body fluids have an unpleasant odour, or develop such odours when in contact with air and/or bacteria for prolonged periods. The literature shows many references relating to odour control in products such as diapers and catamenials. A known solution to this problem is to incorporate an odour control agent in the disposable absorbent article.

In a preferred mode, the odour-control agent is a water-insoluble particulate odour-absorbing material such as chlorophyll particles, activated carbon granules, charcoal, ion exchange resin (Japanese 87019865), activated alumina, and absorbent zeolite materials, including the well known "molecular sieve" zeolites of the type A and X and the zeolite materials marketed under the trade name ABSCENTS by the Union Carbide Corporation and UOP, and which are typically available as a white powder in the 3-5 micron particle size range.

It has now been discovered that the incorporation in a disposable absorbent article of certain preferred odour control agents in particulate form not only provides an advantage in controlling the unpleasant odours related to the body fluids absorbed, but also provides an unexpected benefit in respect to comfort by giving the article a dilatant behaviour, both in the dry and in the wet state, i.e., before and after absorption of body fluids during the use.

Dilatancy is a rheological behaviour of a type of non-Newtonian fluids, accordingly called dilatant fluids, in which the viscosity increases with increasing shear rate. In other words, a dilatant fluid, or, more generically, a dilatant system, opposes an increasing resistance to increasing shear stresses. A well known example of a dilatant material is beach sand, both dry and wet. When considering a dilatant behaviour in a disposable absorbent article, it is meant that the article dynamically changes its physical characteristics by increasing its resistance to deformation and/or its resiliency under increasing stresses during wearing; the article therefore shows a resistance to deformation that overall increases with the amount of deformation. Dilatancy can in fact provide a disposable absorbent article with the capability of better resisting to stresses applied with various speeds during the use, or, alternatively, to prolonged stresses having a relatively high intensity, to prevent bunching and twisting.

A disposable absorbent article provided with a dilatant behaviour has therefore a low resistance to deformation at low levels of stress usually involving a lower shear rate, while it increases its deformation resistance when stresses induced by movements during the wearing time become greater and cause higher shear rates in the article. This results in a better fit during the use and in an increased resistance to bunching and twisting, typically caused by higher levels of stress experienced by the article when worn. The article is moreover more comfortable for the user, since it is capable of following the wearer's movements adapting its deformation resistance to the varying stresses to which it is subjected during the use.

The preferred particulate material incorporated in the disposable absorbent articles of the present invention constitute a dilatant system both in the dry and in the wet state, and this is particularly advantageous since the particulate material can provide the article with dilatant behaviour also in the wet state, i.e. after absorption of body fluid has taken place, when usually the absorbent articles have a lower capacity to withstand twisting and bunching.

### SUMMARY OF THE INVENTION

The present invention relates to the use of a particulate material in a disposable absorbent article for providing the article with dilatant behaviour, in order to provide the article with a better resistance to bunching and twisting in use.

### BRIEF DESCRIPTION OF THE DRAWINGS

While the specification concludes with claims particularly pointing out and distinctly claiming the present invention, it is believed that the present invention will be better understood from the following description in conjunction with the following drawings:
FIG. 1 is a plan view of a sanitary napkin according to the present invention, seen from the side thereof that faces the wearer in use;
FIG. 2 is a graph showing how the force necessary for a deformation of the sanitary napkin varies with the amount of deformation in the dry state;
FIG. 3 is a similar graph showing the same dependency in the wet state.

### DETAILED DESCRIPTION OF THE INVENTION

This invention relates to disposable absorbent articles for application to the user's body, which exhibit absorbency for bodily fluids, the protection of the user's garments from soiling, and improved physical comfort to the user.

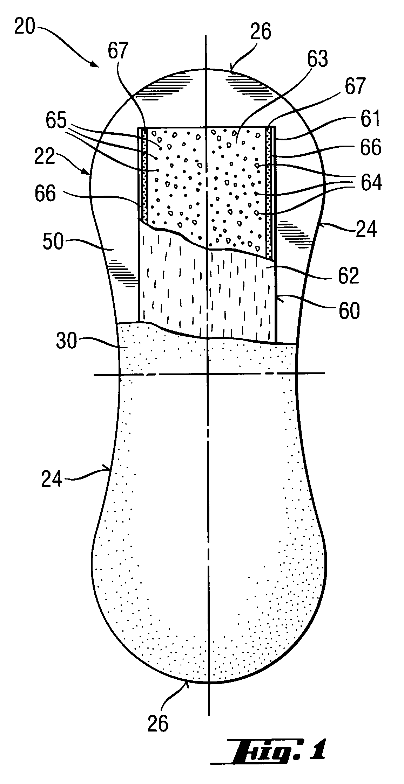

As used herein, the term "absorbent article" refers to devices which absorb and contain body exudates, and, more specifically, refers to devices which are placed against or in proximity to the body of the wearer to absorb and contain the various exudates discharged from the body. The term "disposable" is used herein to describe absorbent articles which are not intended to be laundered or otherwise restored or reused as an absorbent article (i.e., they are intended to be discarded after a single use and, preferably, to be recycled, composted or otherwise disposed of in an environmentally compatible manner). A preferred embodiment of an absorbent article of the present invention is the sanitary napkin 20 shown in Fig. 1, where portions of the structure have been cut-away to more clearly show the construction of the sanitary napkin 20 and with the portion of the sanitary napkin 20 which faces or contacts the wearer oriented towards the viewer. As used herein, the terms "pantiliner", and "sanitary napkin" refer to absorbent articles generally worn by females adjacent to the pudendal region that are intended to absorb and contain the various exudates which are discharged from the body (e.g., vaginal discharges, blood, menses, and urine). The present invention, however, is not limited to the particular types or configurations of absorbent articles shown in the drawing. It should be understood that the present invention is also applicable to other anatomically shaped disposable absorbent articles such as incontinent pads, and the like.

The term "use", as used herein, refers to the period of time that starts when the absorbent article is actually put in contact with the anatomy of the user.

The terms "joined", "affixed" or "secured", as used herein, encompasses configurations whereby a first member is directly connected to a second member and configurations whereby a first member is indirectly connected to a second member by connecting the first member to intermediate members which in turn are connected to the second member.

As used herein, the term "pudendal" refers to the externally visible female genitalia and is limited to the labia majora, the labia minora, the clitoris, and the vestibule.

As shown in Fig. 1, the sanitary napkin 20 comprises an absorbent means represented by main body portion 22. The main body portion 22 has longitudinal edges 24 and lateral edges 26. The term "longitudinal", as used herein, refers to a line, axis or direction in the plane of the sanitary napkin 20 that is generally aligned with (e.g., approximately parallel to) a vertical plane which bisects a standing wearer into left and right body halves when the sanitary napkin 20 is worn. The term "lateral" as used herein, refers to a line, axis or direction which lies within the plane of the sanitary napkin 20 that is generally perpendicular to the longitudinal direction.

The main body portion 22 comprises a topsheet 30, a backsheet 50, and an absorbent core 60.

The topsheet 30 is liquid permeable and when the sanitary napkin 20 is in use, the topsheet 30 is compliant, soft feeling, and non-irritating to the user's skin. Further, the topsheet 30 is liquid pervious, permitting liquid (e.g. menses and/or urine) to readily penetrate through its thickness. A suitable topsheet 30 may be manufactured from a wide range of materials such as woven and nonwoven materials; polymeric materials such as apertured formed thermoplastic films, apertured plastic films, and hydroformed thermoplastic films; porous foams; reticulated foams; reticulated thermoplastic films; and thermoplastic scrims. Suitable woven and nonwoven materials can be comprised of natural fibres (e.g., wood or cotton fibers), synthetic fibres (e.g., polymeric fibres such as polyester, polypropylene, or polyethylene fibres); or from a combination of natural and synthetic fibres.

A preferred topsheet comprises an apertured formed film. Apertured formed films are preferred for the topsheet because they are pervious to body fluids and yet non-absorbent and have a reduced tendency to allow liquids to pass back through and rewet the wearers skin. Thus, the surface of the formed film which is in contact with the body remains dry, thereby reducing body soiling and creating a more comfortable feel for the wearer.

Suitable formed films are described in U.S. Pat. No. 3,929,135, issued to Thompson on December 30, 1975; U.S. Pat. No. 4,324,246, issued to Mullane, et al. on April 13, 1982; U.S. Pat. No. 4,342,314, issued to Radel, et al. on August 3, 1982; U.S. Pat. No. 4,463,045, issued to Ahr, et al. on July 31, 1984; and U.S. Pat. No. 5,006,394, issued to Baird on April 9, 1991. A preferred topsheet for the absorbent article of the present invention is a formed film described in one or more of the above patents and marketed on sanitary napkins by The Procter & Gamble Company of Cincinnati, Ohio as "DRI-WEAVE".

Topsheets having not a homogeneous distribution of liquid passage ways but only a portion of the topsheet comprising liquid passage ways are also contemplated by the present invention. Typically such topsheets would have the liquid passage ways oriented such that they result in a centrally permeable and peripherally impermeable topsheet for liquids.

In a preferred embodiment of the present invention, the body or exposed surface of the formed film topsheet is hydrophilic so as to help liquid transfer through the topsheet faster than if the body surface were not hydrophilic so as to diminish the likelihood that menstrual fluid will flow off the topsheet rather than flowing into and being absorbed by the absorbent core. In a preferred embodiment, surfactant is incorporated into the polymeric materials of the formed film topsheet such as is described in PCT publication WO 93/09741. Alternatively, the body surface of the topsheet can be made hydrophilic by treating it with a surfactant such as is described in U.S. 4,950,254.

Another alternative are so called hybrid topsheets which incorporate fibrous and film like structures particularly useful embodiments of such hybrid topsheets are disclosed in PCT publications WO 93/09744, WO 93/11725 or WO 93/11726.

When referring to the topsheet a multi layer structure or a mono layer structure is contemplated. The hybrid topsheet mentioned above is such a multi layer design but other multi layer topsheets such as primary and secondary topsheet designs are also considered.

The absorbent core 60 is positioned between the topsheet 30 and the backsheet 50. The absorbent core 60 provides the means for absorbing body fluids. The absorbent core 60 need not have an absorbent capacity much greater than the total amount of body fluid anticipated to be absorbed. The total absorbent capacity of the absorbent core should usually be compatible with the design leading and the intended use of the absorbent article. Further, the size and absorbent capacity of the absorbent core may be varied to accommodate different uses. A sanitary napkin as that illustrated in FIG. 1, for example, will have generally larger dimensions, and will comprise an absorbent core 60 with a higher absorbent capacity, as compared to a pantiliner. The absorbent core 60 is generally compressible, conformable, and non-irritating to the user's skin. It can comprise any material used in the art for such purpose. Examples include comminuted wood pulp which is generally referred to as airfelt, creped cellulose wadding, absorbent foams, absorbent sponges, synthetic staple fibres, polymeric fibres, hydrogel-forming polymer absorbent gelling materials, peat moss, or any equivalent material or combinations of materials. The absorbent core may also have a layer of absorbent gelling material, such as in the form of particles, disposed between two air-laid tissue layers (or "upper" and "lower" tissue layers). The first and second tissue layers provide containment of the absorbent gelling material, improved lateral wicking of the absorbed exudates throughout the absorbent core, and a degree of absorbency. Exemplary absorbent structures for use as the absorbent core of the present invention are described in U.S. Patent 4,950,264 entitled "Thin, Flexible Sanitary Napkin" issued to Osborn on August 21, 1990; U.S. Patent 4,610,678 entitled "High-Density Absorbent Structures" issued to Weisman et al. on September 9, 1986; U.S. Patent 4,834,735 entitled "High Density Absorbent Members Having Lower Density and Lower Basis Weight Acquisition Zones", issued to Alemany et al. on May 30, 1989; and European Patent Application No. 0198 683, the Procter & Gamble Company, published October 22, 1986 in the name of Duenk, et al.

In a preferred embodiment of the present invention illustrated in FIG. 1 the absorbent core 60 is constituted by a layered structure in accordance with the disclosure of WO 94/01069 or WO 95/17868, that describe a thin, layered absorbent structure comprising first and second layers 61, 62 of fibrous material, e.g. air laid material, and an intermediate layer 63 comprising particles of absorbent gelling material 64 and particles of a thermoplastic, polymeric material 65; the first and second fibrous layers 61, 62 extend beyond the intermediate layer 63 laterally to form longitudinal edge portions 66. The two fibrous layers 61, 62 are bound together with the intermediate layer 63 between them by the melting of the particles of thermoplastic, polymeric, organic material 65 and by means of a continuous line 67 of adhesive extending longitudinally on each edge portion 66.

WO 95/17868 describes a layered structure substantially as described in WO 94/01069 with the exception that WO 95/17868 comprises a much higher quantity of absorbent gelling material in the intermediate layer which is between the fibrous layers, namely in an amount exceeding 120 g/m².

Absorbent gelling materials mentioned above are those materials which, upon contact with fluids (i.e., liquids) such as water or body fluids, imbibe such fluids and thereby form hydrogels. In this manner, fluids discharged into the absorbent core 60 can be acquired and held by the absorbent gelling materials, thereby providing the articles herein with enhanced absorbent capacity and/or improved fluid retention performance. Preferred absorbent gelling materials which are employed in the absorbent core 60 will generally comprise particles of a substantially water-insoluble, slightly cross-linked, preferably neutralized, hydrogel-forming polymer material. The term "particles", as used herein, can refer to particles in any form, such as in the form of pellets, flakes, or fibres. The characteristics of the absorbent core 60 (including, but not limited to preferred types of polymer materials used herein, and types of methods which can be used for preparing these polymer particles) are described in greater detail in U.S. Patent 5,009,653 issued to Osborn.

The sanitary napkin 20 of the present invention further comprises, e.g. in the absorbent core 60, mixed with the particles of absorbent gelling material 64 and of thermoplastic, polymeric material 65, an odour-control material for controlling unpleasant odours associated with absorbed body fluids.

Any known odour-control agent or any combination thereof that can be suitably included in a disposable absorbent article, including other materials such as binders and/or substrates, can be comprised in the sanitary napkin 20 of the present invention as the odour-control material.

The odour-control material can be incorporated into the sanitary napkin 20 by methods known in the art, for example layered in the intermediate layer 63 of the absorbent core 60.

The absorbent articles according to the present invention comprise a backsheet 50 that is impervious to liquids (e.g. menses, vaginal discharges, and/or urine) and is preferably manufactured from a thin plastic film, although other flexible liquid impervious materials can also be used. In use, the backsheet 50 is interposed between the absorbent core 60 and the user's undergarments. The function of the backsheet 50 is to prevent exudates which may be expelled from or which inadvertently bypass the absorbent core 60 from contacting and soiling the user's undergarments. The backsheet 50 can thus comprise a woven or nonwoven material, polymeric films such as thermoplastic films of polyethylene or polypropylene, or composite materials such as a film-coated nonwoven material. Preferably, the backsheet is a polyethylene film having a thickness of from about 0.012 mm to about 0.015 mm. Exemplary polyethylene films are manufactured by Clopay Corporation of Cincinnati, Ohio, under the designation P18-0401 and by Ethyl Corporation, Visqueen Division, of Terre Haute, Indiana, under the designation XP-39385. The backsheet 50 is preferably embossed and/or matte finished to provide a more clothlike appearance. Further, the backsheet 50 may permit vapours to escape from the absorbent core 60 (i.e., it can be breathable) while still preventing exudates from passing through the backsheet 50.

The backsheet 50 typically extends across the whole of the main body portion 22 of the sanitary napkin 20.

The topsheet, backsheet and absorbent core elements are joined together to provide the sanitary napkin 20 of the present invention. At least two, preferably all of the elements of the sanitary napkin 20 are joined.

Each of said elements comprising at least one layer has a body facing surface and a garment facing surface. Typically, adjacent garment facing surfaces form a common interface with the body facing surface of an adjacent element or layer. The elements or layers are joined together across this common interface. In this manner the topsheet 30 is joined to the absorbent core 60, and the core 60 is joined to the backsheet 50. Furthermore, each of said topsheet 30, backsheet 50 and core 60 elements may comprise more than one layer and these layers may also be similarly joined. In addition the topsheet 30 is directly or indirectly joined to the backsheet 50 at the periphery of the sanitary napkin 20.

The elements and layers thereof may be joined by any means known in the art for affixing two adjacent layers of material, such that the layers are directly attached to one another or directly attached to one another via the joining means. Suitable joining means include adhesive, fusion bonding, ultra sonic bonding, stitching, heat (e.g. crimping), embossing, and/or pressure bonds, or dynamic mechanical bonds. According to an embodiment of the present invention the preferred means of joining is adhesive. Suitable adhesives include non pressure sensitive and cold adhesives. The adhesive may be applied by any means known in the art such as spiral application, slot coating, spraying, spiral spraying, curtain coating, control coating and printing.

In a preferred embodiment of the present invention wherein the absorbent article finds utility as a sanitary napkin 20, the absorbent article is also provided with a panty fastening means which provides means to attach the article to an undergarment. For example the panty fastening means may comprise a mechanical fastener such as hook and loop fasteners such as marketed under the tradename VELCRO, snaps or holders. Alternatively, the article is fastened to the undergarment by means of panty fastening adhesive on the backsheet 50. The panty fastening adhesive provides a means for securing the article to the panty and preferably a means for securing the article when soiled, to the fold and wrap package for convenient disposal. Typically, at least a portion of the garment facing surface of the backsheet 50 is coated with adhesive to form the panty fastening adhesive. Any adhesive or glue used in the art for such purposes can be used for the panty fastening adhesive herein. Pressure sensitive adhesives are most preferred. Suitable adhesives include Century A-305-IV manufactured by the Century Adhesives Corporation of Columbus, Ohio, and Instant LOK 34-2823 manufactured by the National Starch and Chemical Company of Bridgewater, New Jersey, 3 Sigma 3153 manufactured by 3 Sigma and Fuller H-2238ZP manufactured by the H.B. Fuller Co.

The panty fastening adhesive is applied to the backsheet 50 by any means and with any distribution known in the art.

The panty fastening adhesive is typically covered with a removable release paper or film in order to prevent the adhesive from drying out or adhering to another surface other than the panty prior to use. Any commercially available release paper or film may be used. Suitable examples include BL 30MG-A SILOX EI/O and BL 30 MG-A SILOX 4 P/O available from Akrosil Corporation.

According to the present invention, the sanitary napkin 20 is provided with a dilatant behaviour by means of the use of a suitable particulate material incorporated therein. Dilatancy gives the disposable absorbent article of the present invention, e.g. the sanitary napkin 20, the property of increasing its stiffness, i.e. its capability to withstand deformation, when subjected to increasing stresses, involving higher shear rates in the particulate material, caused by the wearers movements during the wearing time. This results in a product that deforms easily and smoothly under stresses involving lower shear rates which usually cause small deformations, therefore being comfortable to the wearer, but is less subject to deformation under higher stresses, that are more likely to cause permanent deformations in a traditional product, e.g. bunching or twisting as a result of compression forces exerted in transverse direction on the sanitary napkin by the wearer's thighs. The article provided with dilatancy is in other words more capable of resisting to stresses applied with various, e.g. increasing, speeds during the use, and also to prolonged stresses having higher intensities.

Bunching and twisting in use usually lead to a sanitary napkin 20 crushed transversely inwardly with its longitudinal edges 24 curled around its longitudinal axis, so that the napkin eventually takes on the appearance of a twisted rope, particularly in its central portion. The phenomenon is in fact known as roping, and causes a reduction of the useful surface of the absorbent article, and therefore of both its absorption capacity and of its capability of properly acquiring the body fluids as they are discharged. The article is also uncomfortable to the wearer since it becomes thicker and is therefore less soft and conformable.

Absorbent articles of thin type, typically having a thickness of less than 5 mm, have a particular tendency to roping when worn, as a result of the squeezing of the article between the thighs of the wearer, which can cause a permanent deformation.

Suitable materials that can be incorporated in a disposable absorbent article according to the present invention in order to provide dilatancy could be in principle those materials, usually in particulate or suspension form, e.g. water suspension, that are known as forming dilatant systems. Suitable materials are typically non swellable particulate materials.

According to the present invention, it has been found that some of the preferred odour control materials that are incorporated in particle form in disposable absorbent articles are not only active in controlling the unpleasant odours associated with the absorbed body fluids, but also involve a further benefit in terms of comfort and effectiveness of the article by providing the disposable absorbent article with the dilatant behaviour, both in dry and in wet state, i.e., before and after absorption of body fluids during the use.

Particularly preferred are odour control substances such as silica, zeolites, including the molecular sieve zeolites of the type A, starch, aluminium oxide (alumina), activated carbon, and mixtures or agglomerates thereof. Quartz powder and polyethylene powder can also be used, even though they alone cannot provide the odour control benefit. Any combination with other known odour control substances, not related to dilatancy, is also possible.

The particulate material for providing dilatancy can be incorporated in different amounts and different particle sizes, and according to that different levels of dilatancy can be achieved, as can determined by the man skilled in the art. The particles can be of any desired suitable shape.

Preferably, the average dimensions of the particulate material used in a disposable absorbent article according to the present invention, given as a weighted average of the smallest dimensions of the individual particles, can be between 50 microns and 1500 microns, preferably between 100 microns and 800 microns.

Preferred odour control materials in particulate form that are also capable of providing an absorbent article such as the sanitary napkin 20 of the present invention with a dilatant behaviour can be among those described in European Patent Applications EP 96109173.3, EP 96109174.1, EP 96109175.8, EP 96109176.6, EP 96109177.4. Most preferred odour control materials in particulate form also providing dilatancy comprise zeolite A and silica gel.

In a preferred embodiment of the present invention, a particulate material 68 comprising zeolite A and silica gel intended to provide odour control and dilatancy is comprised in the intermediate layer 63 of the absorbent core 60, mixed with particles of absorbent gelling material 64. The amounts of the absorbent gelling material particles 64 and of the particulate material for providing odour control and dilatancy can respectively range from about 20 g/m², up to about 600 g/m², and from about 40 g/m² and about 200 g/m².

The dilatant behaviour provided by the particulate material 68 in the sanitary napkin 20 of the present invention can be evaluated, both in the dry and in the wet state, by a compression test performed on the sanitary napkin 20. The test actually measures the force necessary to deform, i.e., to bend, in longitudinal direction a sanitary napkin 20 featuring a dilatant behaviour, as a function of the amount of the deformation induced at constant speed; the test therefore measures the resistance of the sanitary napkin to forces directed transversely, as those exerted in use by the inner part of the wearer's thighs. The results have been compared to those obtained with a reference sample comprising no particulate material for providing dilatancy.

### Samples

Both the reference sample and the sample according to the present invention used in the compression test were sanitary napkins of the type Always Ultra currently available on the market, having the absorbent core replaced by a layered structure 70 mm wide made according to the disclosure of WO 94/01069. The layered structure incorporated in the reference sample comprises a first and second fibrous layer, each constituted by an airlaid nonwoven web having a basis weight of 60 g/m², and an intermediate layer comprising particles of absorbent gelling material in an amount of 63.80 g/m². The layered core structure of the reference sample is currently sold by Korma, Italy, under the code XA07001001. The layered structure comprised in the sanitary napkin according to the present invention differs form the layered structure of the reference sample in that it further comprises in the intermediate layer particles of zeolite A in an amount of 61.50 g/m², and particles of silica gel in an amount of 86.37 g/m² in order to provide dilatancy, homogeneously mixed with the particles of absorbent gelling material. The release paper is removed from each sample and the panty fastening adhesive is neutralized with talc. Each side flap is cut with scissors along a line parallel to the respective longitudinal edge of the absorbent core.

### Compression test, dry

The apparatus used for the test is an INSTRON 6021 Dynamometer (Tensile Tester), with the load cell calibration set at 10 N; the compression speed is set at 100 mm/min and the Tensile Tester is set to run a Compression test; the clamp distance is set at 65 mm. Each sample is positioned symmetrically between the fixed and the moving clamp, with each clamp holding a portion of the absorbent core 2.5 mm wide along the respective longitudinal edge, and with the topsheet facing the operator. The initial force exerted on the sample is less than 0.100 N. The sample is slightly biased in order to have it bend in longitudinal direction during compression with the convexity on the topsheet side.

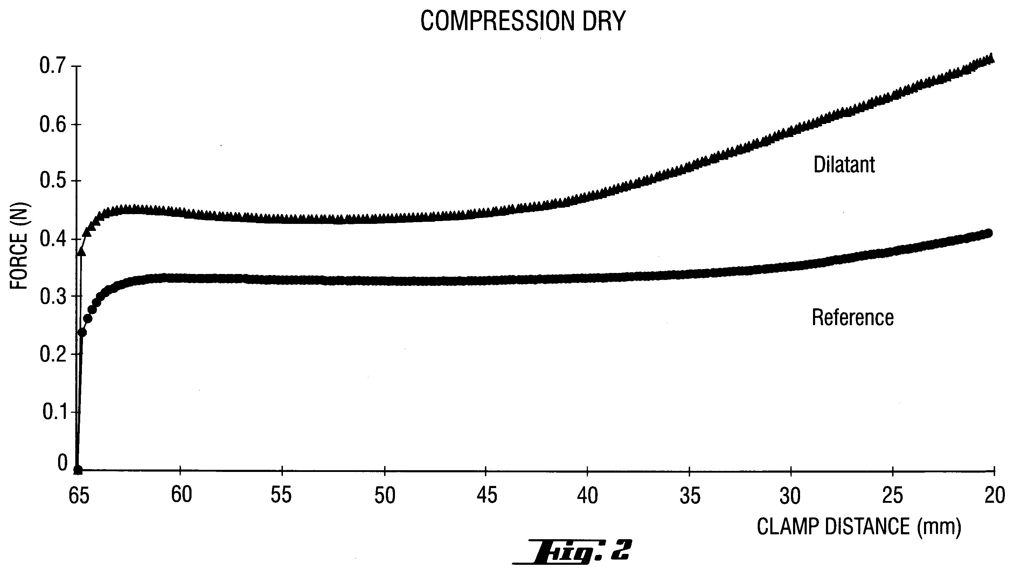

The test is run and the force in N exerted by the sample on the moving clamp is recorded on a graph, shown in FIG. 2, as a function of the sample deformation, i.e., of the distance between the clamps.

### Compression test, wet

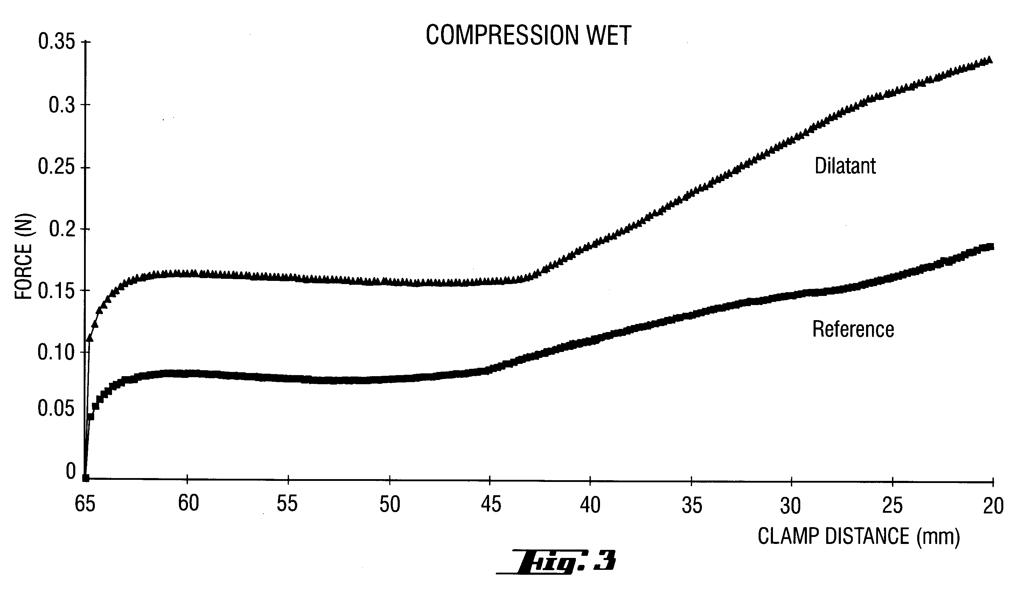

After the compression test in dry conditions the same sample is removed from the clamps of the tester and put onto a flat plate; 12 ml of PIF fluid are released onto the centre of the product at a flow rate of 8 ml/min, and the sample is left on the plate for 20 minutes. Then the sample is compressed for 15 sec with a weight exerting a pressure of 70 g/cm² on a surface of 4.5x10 cm centered on the sample itself, and the compression test is repeated with the same procedure as described for the dry state. The force is recorded on the similar graph of FIG. 3 as a function of the deformation.

In both graphs of FIGS. 2 and 3 the reference sample has been identified as "Reference", while the sample according to the present invention has been identified as "Dilatant".

### Preparation of Test Solution Paper Industry Fluid (PIF)

The test solution PIF is a widely used test liquid in the Paper industry due to its simple composition, ability to prepare and maintain high standards of solution quality and its similarity to human menses with respect to viscosity and ionic surface tension.

The solution PIF is prepared by dissolving the following reagent components, at the indicated quantities, into 1 litre of distilled water. Care should be taken in dissolving the solid components and particularly the Carboxylmethylcellulose. Typically the solid components should be added over a period of one hour slowly and with constant stirring of the solution (via a magnetic stirring device).
Supplier Sigma Chemicals, USA

| Chemical Component | | Usage / 1L |
|---|---|---|
| 1) | Carboxymethylcellulose, Sodium salt low viscosity: Order No. = C 5678, | 15 grams |
| 2) | Sodium bi-Carbonate, Crystalline: Order No. = S 8875 | 4 grams |
| 3) | Sodium Chloride (AR): Order No. = S 9625 | 10 grams |
| 4) | Glycerol (>99% pure): Order No. = G 5516 | 80 grams |

The results of the Compression test in dry and wet conditions are illustrated in the two graphs of FIGS. 2 and 3, respectively.

In both graphs the force opposed by the dilatant sanitary napkin at very low compressions, where the dilatant behaviour is much less evident, is higher as compared to the force opposed by the reference sample, because the dilatant sample comprises a higher basis weight of particulate material as compared to the reference sample, and therefore possibly the bonding action performed by the melting of the thermoplastic, polymeric, organic material particles is proportionally higher, since the amount of the melting particles is a given percentage of the total amount of the mixture.

Anyway, FIGS. 2 and 3 show how the force opposed to the compression in transverse direction (deformation along a longitudinal direction) increases with the increasing deformation much more in the sanitary napkin provided with the dilatant behaviour as compared to the reference sample, both in the dry and in the wet state. For example, the difference between the forces exerted by the dilatant sanitary napkin and by the reference sample in correspondence of a distance between the clamps of 20 mm, is about twice as much the same difference at a deformation of 10 mm. Of course the absolute values of the force are on average lower in the wet state, where the strength of the structure against deformation is overall lower.

The test demonstrates that the dilatant behaviour provided by the preferred odour control particulate material gives the product a better capacity to withstand permanent deformations induced by increasing forces exerted by the body as it moves during the wearing time, not only in the dry state of the article, but also in the wet state, i.e., after absorption of fluid, when usually the structures of disposable absorbent articles become weaker and less resistant to twisting and bunching (roping).

The particulate material capable of providing the disposable absorbent article of the present invention with the dilatant behaviour can be alternatively comprised in any other location within the absorbent article, other than in the absorbent core, but preferably in a position that allows the particulate material to come in contact with body fluid during the use.

Without being bound to any theory, it is in fact believed that when the liquid contacts the particulate material this creates a dilatant suspension and a more effective dilatant system is provided.

Although the present invention has been so far described in the context of substantially planar disposable absorbent articles, it can be alternatively applied to tridimensionally shaped disposable absorbent articles, both those having a pre formed shape prior to use, and those that are capable of shaping and forming in use.

In an alternate embodiment of the present invention, the disposable absorbent article may have two flaps (not shown), each of which is adjacent to and extends laterally from the respective side edge of the absorbent core. The flaps are configured to drape over the edges of the wearer's panties in the crotch region so that the flaps are disposed between the edges of the wearer's panties and the wearer's thighs. The flaps help serve to prevent soiling of the wearer's body and panties by menstrual fluid, preferably by forming a double wall barrier along the edges of the panty. The flaps may be also provided with attachment means on their garment facing surface so that the flaps can be folded back under the panty and attached to the garment facing side of the panty.

The flaps may be constructed of various materials including materials used for the topsheet 30, backsheet 50, combinations thereof, and may be a laminate having tissue in the centre. Further, the flaps may be a separate element attached to the main body of the tridimensional absorbent article or can comprise extensions of the topsheet 30 and/or backsheet 50. It is recommended, however, that the flaps have a liquid impervious backsheet to prevent body fluids which reach the flaps from soiling the edges of the wearer's panties. Even though the area of the flaps can be considered as portions of the article comprising at least the topsheet and the backsheet extending beyond the absorbent core, it is generally meant that the flaps do not constitutes lobes, in the sense intended according to the present invention.

Preferred flaps that are suitable or adaptable to the tridimensional absorbent article of the present invention are disclosed in U.S. Pat. No. 4,687,478 issued to Van Tilburg on Aug. 18, 1987; U.S. Pat. No. 4,589,876 issued to Van Tilburg on May 20, 1986; and U.S. Pat No. 4,608,047 issued to Mattingly on Aug. 26, 1986.

Optionally, the absorbent article may comprise components that naturally wrap the sides of a wearer's panties. Sanitary napkins having components that naturally wrap the sides of a wearer's panties suitable for use with the tridimensional absorbent article of the present invention are disclosed in U.S. Patent No. 5,584,829 entitled "Absorbent Article having Panty Covering Components that Naturally Wrap the Sides of Panties", issued to Lavash, et al. on Dec. 17, 1996, and U.S. Patent No. 5,558,663 entitled "Absorbent Articles Having Undergarment Covering Components with Zones of Extensibility", issued to Weinberger, et al. on Sep. 24, 1996.

Although the anatomically shaped disposable absorbent article of the present invention has been described with reference to a sanitary napkin, it can be used beneficially in the context of other disposable absorbent articles such as sanitary napkins and incontinence articles. The disposable absorbent article may thus also have all those features and parts which are typical for products in the context of their intended use.

## Claims

1. Use of particulate material in a disposable absorbent article for providing said article with dilatant behaviour in order to provide said article with a better resistance to bunching and twisting in use

2. Use according to claim 1, characterized in that said disposable absorbent article comprises a liquid pervious topsheet, a liquid impervious backsheet joined to said topsheet, and an absorbent element intermediate said topsheet and said backsheet.

3. Use according to claim 2, characterized in that said particulate material is comprised within said absorbent element.

4. Use according to any preceding claim, characterized in that said particles have an average dimension between 50 microns and 1500 microns, preferably between 100 microns and 800 microns.

5. Use according to any preceding claim, characterized in that said particulate material is a non swellable particulate material.

6. Use according to any preceding claim, characterized in that said particulate material is an odour control material.

7. Use according to any preceding claim, characterized in that said particulate material comprises zeolite and silica particles.
